# FASCICULE DE BREVET EUROPEEN

(11) **EP 1 655 030 B1**
(45) Date de publication et mention de la délivrance du brevet: **19.12.2007**
(21) Numéro de dépôt: 05292310.9
(22) Date de dépôt: 02.11.2005
(51) Int. Cl.: A61K 31/5415, C07D 285/24, A61P 25/00

(54) **Dérivés de benzothiadiazine, leur procédé de préparation et les compositions pharmaceutiques qui les contiennent**
Benzothiadiazinderivate, Verfahren zu deren Herstellung und pharmazeutische Zusammensetzungen, die diese enthalten
Benzothiadiazine derivatives, method for preparing same and pharmaceutical compositions containing same

(30) Priorité: 03.11.2004 FR 0411690
(43) Date de publication de la demande: 10.05.2006
(73) Titulaire: Les Laboratoires Servier, 92415 Courbevoie Cedex (FR)
(72) Inventeur: Desos, Patrice, 92270 Bois-Colombes (FR); Cordi, Alexis, 92150 Suresnes (FR); Lestage, Pierre, 78170 La Celle-St-Cloud (FR)

(56) Documents cités:
- WO-A-01/40210
- WO-A-98/12185
- WO-A-03/053947
- WO-A-03/053979

## Description

La présente invention concerne de nouveaux dérivés de benzothiadiazine, leur procédé de préparation et les compositions pharmaceutiques qui les contiennent.

Il est désormais reconnu que les aminoacides excitateurs, et tout particulièrement le glutamate, jouent un rôle crucial dans les processus physiologiques de plasticité neuronale et dans les mécanismes sous-tendant l'apprentissage et la mémoire. Des études pathophysiologiques ont indiqué clairement qu'un déficit de la neurotransmission glutamatergique est associé étroitement avec le développement de la maladie d'Alzheimer (Neuroscience and Biobehavioral reviews, 1992, 16, 13-24 ; Progress in Neurobiology, 1992, 39, 517-545).

Par ailleurs, d'innombrables travaux ont démontré, durant les dernières années, l'existence de sous-types réceptoriels aux aminoacides excitateurs et de leurs interactions fonctionnelles (Molecular Neuropharmacology, 1992, 2, 15-31).

Parmi ces récepteurs, le récepteur à l'AMPA ("α-amino-3-hydroxy-5-methyl-4-isoxazole-propionic acid") apparaît être le plus impliqué dans les phénomènes d'excitabilité neuronale physiologique et notamment dans ceux impliqués dans les processus de mémorisation. Pour exemple, l'apprentissage a été montré comme étant associé à l'augmentation de la liaison de l'AMPA à son récepteur dans l'hippocampe, l'une des zones cérébrales essentielles aux processus mnémocognitifs. De même, les agents nootropes, tels que l'aniracetam, ont été décrits très récemment comme modulant positivement les récepteurs AMPA des cellules neuronales (Journal ofNeurochemistry, 1992, 58, 1199-1204).

Dans la littérature, des composés de structure benzamide ont été décrits pour posséder ce même mécanisme d'action et pour améliorer les performances mnésiques (Synapse, 1993, 15, 326-329). Le composé BA 74, en particulier, est le plus actif parmi ces nouveaux agents pharmacologiques.

Enfin, le brevet EP 692 484 décrit un dérivé de benzothiadiazine possédant une activité facilitatrice sur le courant AMPA et la demande de brevet WO 99/42456 décrit, entre autres, certains dérivés de benzothiadiazine en tant que modulateurs des récepteurs AMPA.

Les dérivés de benzothiadiazine, objets de la présente invention, outre le fait qu'ils soient nouveaux, présentent, de manière surprenante, des activités pharmacologiques sur le courant AMPA particulièrement intéressantes. Ils sont utiles en tant que modulateurs AMPA pour le traitement ou la prévention des désordres mnémocognitifs associés à l'âge, aux syndromes anxieux ou dépressifs, aux maladies neurodégénératives progressives, à la maladie d'Alzheimer, à la maladie de Pick, à la chorée d'Huntington, à la schizophrénie, aux séquelles des maladies neurodégénératives aiguës, aux séquelles de l'ischémie et aux séquelles de l'épilepsie.

Plus spécifiquement, la présente invention concerne les composés de formule (**I**) : 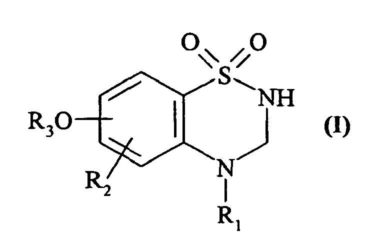 dans laquelle :
- **R₁**: représente un groupement alkyle (C₁-C₆) linéaire ou ramifié substitué par un ou plusieurs atomes d'halogène,
- **R₂**: représente un atome d'hydrogène ou d'halogène ou un groupement hydroxy,
- **R₃**: représente un groupement aryle non substitué ou un groupement aryle substitué par un
ou plusieurs groupements, identiques ou différents, choisis parmi :
alkyle (C₁-C₆) linéaire ou ramifié; alkoxy (C₁-C₆) linéaire ou ramifié; polyhalogénoalkyle (C₁-C₆) linéaire ou ramifié ; atomes d'halogène ; alkoxycarbonyle (C₁-C₆) linéaire ou ramifié ; alkylthio (C₁-C₆) linéaire ou ramifié ; carboxy ; acyle (C₁-C₆) linéaire ou ramifié ; polyhalogénoalkoxy (C₁-C₆) linéaire ou ramifié ; hydroxy ; cyano ; nitro ; amidino (éventuellement substitué par un ou deux groupements identiques ou différents choisis parmi alkyle (C₁-C₆) linéaire ou ramifié, hydroxy, alkoxy (C₁-C₆)
linéaire ou ramifié, et 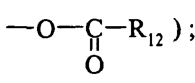 amino (éventuellement substitué par un ou deux groupements alkyle (C₁-C₆) linéaire ou ramifié) ; aminocarbonyle (éventuellement substitué par un ou deux groupements alkyle (C₁-C₆) linéaire ou ramifié) ; benzyloxy ; alkyl (C₁-C₆) sulfonylamino (éventuellement substitué sur l'azote par un groupement (C₁-C₆) linéaire ou ramifié) ; (trifluorométhylsulfonyl)amino ; groupement hétérocyclique ; et alkyle (C₁-C₆) linéaire ou ramifié substitué d'une part par un ou plusieurs groupements, identiques ou différents, choisis parmi les atomes d'hydrogène ou d'halogène et le groupement alkyle (C₁-C₆) linéaire ou ramifié, et substitué d'autre part par un groupement choisi parmi NR₄R₅, S(O)ₙR₆, OR₇, amidino (éventuellement substitué par un ou deux groupements identiques ou différents choisis parmi alkyle (C₁-C₆) linéaire ou ramifié, hydroxy, alkoxy (C₁-C₆) linéaire ou ramifié, et 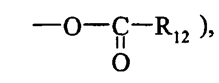 et groupement hétérocyclique, dans lesquels :
R₄ représente un atome d'hydrogène, ou un groupement alkyle (C₁-C₆) linéaire ou ramifié, S(O)ₚR₈, COR₉ ou P(O)(OR₁₀)(OR₁₁),
R₅ représente un atome d'hydrogène, ou un groupement alkyle (C₁-C₆) linéaire ou ramifié,
ou bien R₄ et R₅ forment ensemble, avec l'atome d'azote qui les porte, un groupement hétérocyclique,
R₆, R₈, R₉, R₁₀, R₁₁ et R₁₂ identiques ou différents, représentent un atome d'hydrogène ou un groupement alkyle (C₁-C₆) linéaire ou ramifié éventuellement substitué par un ou plusieurs atomes d'halogène ; un groupement arylalkyle (C₁-C₆) linéaire ou ramifié ; ou un groupement aryle,
R₇ représente un groupement alkyle (C₁-C₆) linéaire ou ramifié ou acyle (C₁-C₆) linéaire ou ramifié,
n et p, identiques ou différents, représentent 0, 1 ou 2,
leur énantiomères et diastéréoisomères ainsi que leurs sels d'addition à un acide ou a une base pharmaceutiquement acceptable,
étant entendu que :
✦ par groupement hétérocyclique, on comprend groupement aromatique ou non, monocyclique ou bicyclique, contenant un à quatre hétéroatomes, identiques ou différents, choisis parmi azote, oxygène et soufre, éventuellement substitué par un ou plusieurs groupements, identiques ou différents, choisis parmi halogène, alkyle (C₁-C₆) linéaire ou ramifié, alkoxy (C₁-C₆) linéaire ou ramifié, polyhalogénoalkyle (C₁-C₆) linéaire ou ramifié, alkoxycarbonyle (C₁-C₆) linéaire ou ramifié, oxo, thioxo, carboxy, acyle (C₁-C₆) linéaire ou ramifié, polyhalogénoalkoxy (C₁-C₆) linéaire ou ramifié, hydroxy, cyano, nitro, amino (éventuellement substitué par un ou plusieurs groupements alkyle (C₁-C₆) linéaire ou ramifié), aminosulfonyle (éventuellement substitué par un ou plusieurs groupements alkyle (C₁-C₆) linéaire ou ramifié), et alkyl (C₁-C₆) sulfonylamino,
✦ par groupement aryle, on comprend groupement aromatique monocyclique ou un groupement bicyclique dans lequel au moins un des cycles est aromatique, éventuellement substitué par un ou plusieurs groupements, identiques ou différents, choisis parmi halogène, alkyle (C₁-C₆) linéaire ou ramifié (éventuellement substitué par un ou plusieurs groupements hydroxy), alkoxy (C₁-C₆) linéaire ou ramifié, polyhalogénoalkyle (C₁-C₆) linéaire ou ramifié, alkoxy carbonyle (C₁-C₆) linéaire ou ramifié, oxo, thioxo, alkylthio (C₁-C₆) linéaire ou ramifié, carboxy, acyle (C₁-C₆) linéaire ou ramifié, polyhalogénoalkoxy (C₁-C₆) linéaire ou ramifié, hydroxy, cyano, nitro, amino (éventuellement substitué par un ou plusieurs groupements alkyle (C₁-C₆) linéaire ou ramifié ou acyle (C₁-C₆) linéaire ou ramifié), aminocarbonyle (éventuellement substitué par un ou plusieurs groupements alkyle (C₁-C₆) linéaire ou ramifié), aminosulfonyle (éventuellement substitué par un ou plusieurs groupements alkyle (C₁-C₆) linéaire ou ramifié), mono ou di(alkyl(C₁-C₆)sulfonyl)amino, mono ou di(trifluorométhylsulfonyl)amino, PO(ORₐ)(OR_{b}) (dans lequel Rₐ, R_{b}, identiques ou différents, représentent un atome d'hydrogène ou un groupement alkyle (C₁-C₆) linéaire ou ramifié), benzyloxy et phényle (éventuellement substitué par un ou plusieurs groupements, identiques ou différents, choisis parmi halogène, alkyle (C₁-C₆) linéaire ou ramifié, perhalogénoalkyle (C₁-C₆) linéaire ou ramifié, hydroxy et alkoxy (C₁-C₆) linéaire ou ramifié).

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, etc.

Parmi les bases pharmaceutiquement acceptables, on peut citer à titre non limitatif l'hydroxyde de sodium, l'hydroxyde de potassium, la triéthylamine, la tertbutylamine, etc.

Le groupement R₁ préféré est l'halogénoéthyle comme le fluoroéthyle, le chloroéthyle, le bromoéthyle, et plus préférentiellement le fluoro ou chloroéthyle.

R₂ représente préférentiellement un atome d'hydrogène.

Le groupement R₃ préféré est le groupement phényle ou phényle substitué, et plus particulièrement substitué par le groupement :
- amidino,
- hydroxyamidino,
- alkoxy,
- alkylsulfonylamino éventuellement substitué sur l'azote par un groupement alkyle,
- ou alkyle substitué par un groupement amidino, hydroxyamidino, OR₇, NHS(O)ₚR₈ ou NHCOR₉.

Encore plus particulièrement, l'invention concerne les composés de formule (I) qui sont :
- le *N*-(4-{[4-(2-bromoéthyl)-1,1-dioxido-3,4-dihydro-2*H*-1,2,4-benzothiadiazin-7-yl]oxy}benzyl)méthanesulfonamide,
- le *N*-(4-{[4-(2-fluoroéthyl)-1,1-dioxido-3,4-dihydro-2*H*-1,2,4-benzothiadiazin-7-yl]oxy} benzyl)méthanesulfonamide,
- le *N*-(4-{[4-(2-chloroéthyl)-1,1-dioxido-3,4-dihydro-2*H*-1,2,4-benzothiadiazin-7-yl]oxy}benzyl)méthanesulfonamide,
- le *N*-(3-{[4-(2-fluoroéthyl)-1,1-dioxido-3,4-dihydro-2*H*-1,2,4-benzothiadiazin-7-yl]oxy}phényl)méthanesulfonamide,
- le *N*-(4-{[4-(2-fluoroéthyl)-1,1-dioxido-3,4-dihydro-2*H*-1,2,4-benzothiadiazin-7-yl]oxy} phényl)méthanesulfonamide,
- le 4-(2-fluoroéthyl)-7-(3-méthoxyphénoxy)-3,4-dihydro-2*H*-1,2,4-benzothiadiazine 1,1-dioxide,
- le *N*-(3-{[4-(2-fluoroéthyl)-1,1-dioxido-3,4-dihydro-2*H*-1,2,4-benzothiadiazin-7-yl]oxy} benzyl)acétamide,
- le *N*-(3-([4-(2-fluoroéthyl)-1,1-dioxido-3 ,4-dihydro-2*H*-1,2,4-benzothiadiazin-7-yl]oxy} benzyl)méthanesulfonamide,
- le *N*-(4-{[4-(2-fluoroéthyl)-1,1-dioxido-3,4-dihydro-2*H*-1,2,4-benzothiadiazin-7-yl]oxy} benzyl)-*N*-méthylméthanesulfonamide,
- le 4-(2-fluoroéthyl)-7-phénoxy-3,4-dihydro-2*H*-1,2,4-benzothiadiazine 1,1-dioxide,
- le 3-{[4-(2-fluoroéthyl)-1,1-dioxido-3,4-dihydro-2*H*-1,2,4-benzothiadiazin-7-yl]oxy}-N'-hydroxybenzènecarboximidamide,
- et le 3-{[4-(2-fluoroéthyl)-1,1-dioxido-3,4-dihydro-2*H*-1,2,4-benzothiadiazin-7-yl]oxy}-*N*-méthylbenzamide.

L'invention concerne également le procédé de préparation des composés de formule (I) caractérisé en ce que l'on utilise comme produit de départ le composé de formule (II) : 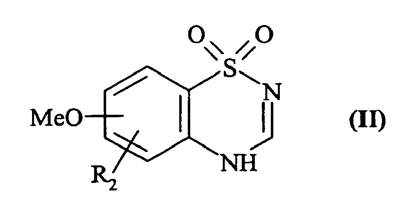 dans laquelle R₂ est tel que défini dans la formule (I),
sur lequel on condense, en milieu basique, un halogénoalkyle (C₁-C₆) linéaire ou ramifié comportant une fonction hydroxyle,
que l'on transforme ensuite en dérivé halogéné correspondant pour conduire au composé de formule (III) : 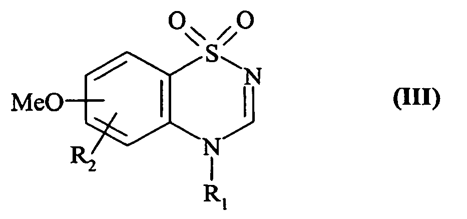 dans laquelle R₁ et R₂ sont tels que définis dans la formule (I),
➢ que l'on soumet à une réaction de déméthylation, en présence de BBr₃ ou BF₃ par exemple, pour conduire au composé de formule (IV) : 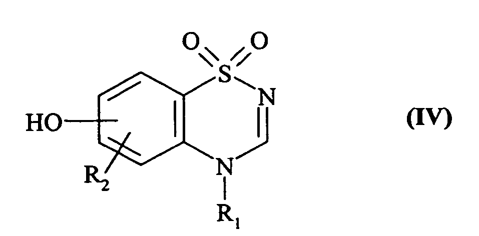 dans laquelle R₁ et R₂ sont tels que définis précédemment,
sur lequel on condense, en présence de Cu(OAc)₂, le dérivé boronique de formule (V) :

R₃-B(OH)₂ (V)

dans laquelle R₃ est tel que défini dans la formule (I),
pour conduire au composé de formule (VI) : 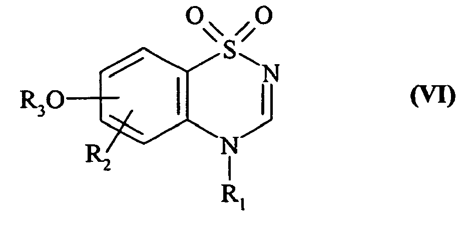 dans laquelle R₁, R₂ et R₃ sont tels que définis précédemment,
que l'on soumet à une réduction avec NaBH₄ par exemple, pour conduire au composé de formule (I) : 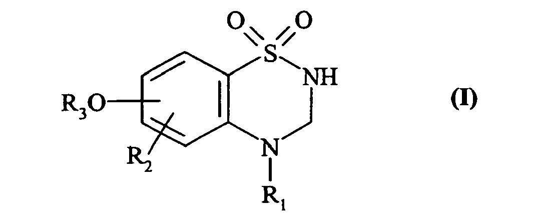 dans laquelle R₁, R₂ et R₃ sont tels que définis précédemment,
➢ ou composé et formule (III) que l'on soumet à une réduction, en présence de NaBH₄ par exemple, pour obtenir le composé de formule (VII) : 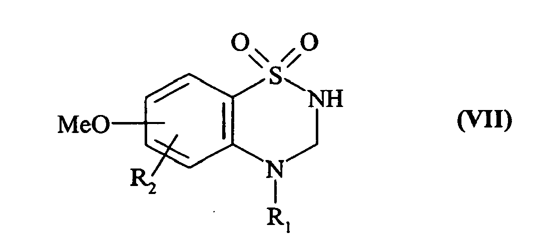 dans laquelle R₁ et R₂ sont tels que définis précédemment,
que l'on soumet à une réaction de déméthylation, en présence de BBr₃ ou BF₃ par exemple, pour conduire au composé de formule (VIII) : 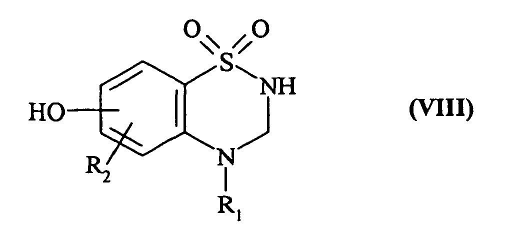 dans laquelle R₁ et R₂ sont tels que définis précédemment,
sur lequel on condense, en présence de Cu(OAc)₂, le dérivé boronique de formule (V) tel que défini précédemment pour conduire au composé et formule (I),
composé de formule (I) que l'on purifie, le cas échéant, selon une technique classique de purification, dont on sépare éventuellement les isomères, selon une technique classique de séparation, et que l'on transforme, si on le souhaite, en leurs sels d'addition, à un acide ou à une base pharmaceutiquement acceptable.

L'invention concerne également le composé de formule (VIII) : 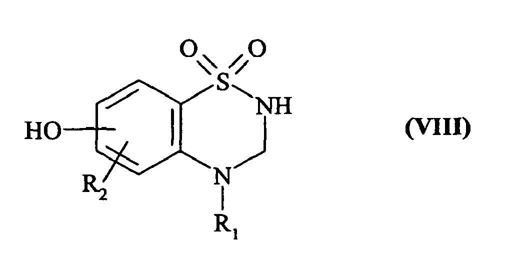 tel que défini précédemment, utile en tant qu'intermédiaire de synthèse pour la synthèse des composés de formules (I) et utile en tant qu'agent modulateur des récepteurs AMPA,
et plus particulièrement le composé de formule (IX), cas particulier des composés de formule (VIII) : 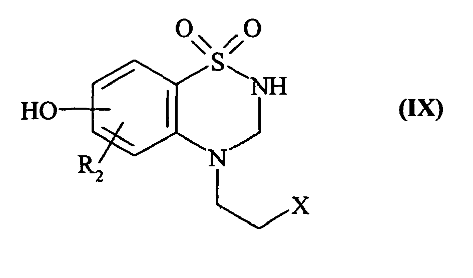 dans laquelle X représente un atome de fluor, de chlore, de brome ou d'iode utile en tant qu'intermédiaire de synthèse pour la synthèse des composés de formules (I) et utile en tant qu'agent modulateur des récepteurs AMPA.

L'invention s'étend aussi aux compositions pharmaceutiques renfermant comme principe actif un composé de formule (I), (VIII) ou (IX) avec un ou plusieurs excipients inertes, non toxiques et appropriés. Parmi les compositions pharmaceutiques, selon l'invention, on pourra citer plus particulièrement celles qui conviennent pour l'administration orale, parentérale (intraveineuse ou sous-cutanées), nasale, les comprimés simples ou dragéifiés, les comprimés sublinguaux, les gélules, les tablettes, les suppositoires, les crèmes, les pommades, les gels dermiques, les préparations injectables, les suspensions buvables, etc.

La posologie utile est adaptable selon la nature et la sévérité de l'affection, la voie d'administration ainsi que l'âge et le poids du patient. Cette posologie varie de 1 à 500 mg par jour en une ou plusieurs prises.

Les préparations et exemples suivants illustrent l'invention mais ne la limitent en aucune façon.

Les produits de départ utilisés sont des produits connus ou préparés selon des modes opératoires connus.

Les structures des composés, décrits dans les exemple, ont été déterminées selon les techniques spectrophotométriques usuelles (infrarouge, RMN, spectrométrie de masse...).

### Préparation 1 : 4-(2-Bromoéthyl)-3,4-dihydro-2H-1,2,4-benzothiadiazin-7-ol 1,1-dioxide

### Stade A : 2-(7-Méthoxy-1,1-dioxido-4H-1,2,4-benzothiadiazin-4-yl)éthanol

A une solution de 7-méthoxy-4*H*-1,2,4-benzothiadiazine 1,1-dioxide (4,0 g, 18,8 mmol) dans un mélange de 30 ml de DMF et 30 ml de CH₃CN, on ajoute 8,6 g (56,6 mmol) de CsF et 1,47 ml (18,8 mmol) de 2-bromoéthanol. On agite 2 h à 75 °C et on rajoute 1,47 ml (18,8 mmol) de 2-bromoéthanol. Après 6 h supplémentaires à 75 °C, on rajoute à nouveau 1,47 ml (18,8 mmol) de 2-bromoéthanol puis 2,8 g (18,8 mmol) de CsF et poursuit l'agitation à 75 °C pendant une nuit. On filtre les sels à température ambiante, rince avec du CH₃CN, évapore le filtrat à sec . On reprend le résidu dans CH₂Cl₂, lave la phase organique avec du NaCl saturé, sèche (MgSO₄). Après évaporation, le résidu collant est repris dans un mélange éther éthylique / CH₂Cl₂. On triture la gomme jusqu'à obtention d'un solide que l'on filtre pour obtenir le produit du titre.

### Point de fusion : 160-162 °C

| *Microanalyse élémentaire :* | | | | |
|---|---|---|---|---|
| | *C* | *H* | *N* | *S* |
| *% théorique* | *46,87* | *4,72* | *10,93* | *12,51* |
| *% expérimental* | *46,99* | *4,96* | *10,34* | *12,51* |

### Stade B : 4-(2-Fluoroéthyl)-7-méthoxy-4H-1,2,4-benzothiadiazine 1,1-dioxide

A une solution de 3,85 g (15,02 mmol) du produit du stade précédent dans 100 ml de CH₂Cl₂ refroidis dans un bain de glace, on rajoute goutte à goutte 3,97 ml (30,0 mmol) de DAST dilué dans 20 ml de CH₂Cl₂. On laisse ensuite la solution réactionnelle revenir à température ambiante en 1 h environ puis on verse 100 ml de NaCl saturée, décante la phase organique, sèche (MgSO₄), évapore sous vide. Le résidu est trituré dans un mélange éther éthylique/ CH₂Cl₂ jusqu'à obtention d'un solide que l'on filtre pour obtenir le produit du titre.

### Point de fusion : 123-128 °C

| *Microanalyse élémentaire :* | | | | |
|---|---|---|---|---|
| | *C* | *H* | *N* | *S* |
| *% théorique* | *46,50* | *4,29* | *10,85* | *12,42* |
| *% expérimental* | *45,88* | *4,41* | *10,46* | *12, 61* |

### Stade C : 4-(2-Fluoroéthyl)-7-méthoxy-3,4-dihydro-2H-1,2,4-benzothiadiazine 1,1-dioxide

A une suspension de 2,77 g (10,7 mmol) du produit du stade précédent dans 25 ml d'éthanol, on rajoute par petites portions, 454 mg (12,0 mmol) de NaBH₄. Après 2 h d'agitation à température ambiante, on ajoute, goutte à goutte, de l'HCl 1N jusqu'à formation d'un précipité blanc que l'on filtre pour récupérer le produit du titre.

### Point de fusion : 91-93 °C

### Stade D : 4-(2-Bromoéthyl)-3,4-dihydro-2H-1,2,4-benzothiadiazin-7-ol 1,1-dioxide

A une solution de 1,53 g (5,88 mmol) du produit du stade précédent dans 70 ml de CH₂Cl₂ refroidis dans un bain de glace, on ajoute, goutte à goutte, 17,6 ml (17,6 mmol) d'une solution 1M de BBr₃ dans CH₂Cl₂. On agite une nuit en laissant revenir à température ambiante. La suspension réactionnelle est refroidie dans un bain de glace et on ajoute, goutte à goutte, 50 ml d'eau. Après 30 min d'agitation, on filtre le précipité, le rince à l'eau et le sèche sous vide. On obtient ainsi le produit attendu sous forme d'une poudre marron clair.

### Point de fusion : 144-148 °C

### Préparation 2 : 4-(2-Fluoroéthyl)-3,4-dihydro-2H-1,2,4-benzothiadiazin-7-ol 1,1-dioxide

### Stade A : 4-(2-Fluoroéthyl)-4H-1,2,4-benzothiadiazin-7-ol 1,1-dioxide

Dans un bicol purgé à l'azote et connecté à un piège contenant de l'hypochlorite de sodium, on introduit, au moyen d'une canule et en poussant à l'azote, 100 ml (950 mmol) du complexe BF₃ Me₃S. Sous agitation et léger courant d'azote, on additionne ensuite rapidement par petites portions une suspension de 5,63 g (21,8 mmol) du produit du stade B de la préparation I dans 75 ml de CH₂Cl₂. On stoppe le courant d'azote et agite la suspension réactionnelle une nuit à température ambiante. Le milieu réactionnel est refroidi dans un bain de glace et on y ajoute de la glace et de l'eau. La suspension est agitée 30 min, le précipité est filtré, rincé à l'eau et à l'heptane. Le solide est séché et recristallisé dans l'eau pour conduire au produit du titre.

### Point de fusion : 230-235 °C

| *Microanalyse élémentaire :* | | | | |
|---|---|---|---|---|
| | *C* | *H* | *N* | *S* |
| *% théorique* | *44,26* | *3,71* | *11,47* | *13,13* |
| *% expérimental* | *44,55* | *4,18* | *11,34* | *13,59* |

### Stade B : 4-(2-Fluoroéthyl)-3,4-dihydro-2H-1,2,4-benzothiadiazin-7-ol 1,1-dioxide

On procède, comme dans le stade C de la préparation 1, à partir du composé obtenu au stade A sauf que le produit du titre ne précipite pas après addition de HCl 1N mais est extrait par du CH₂Cl₂.

### Point de fusion : 178-180 °C

| *Microanalyse élémentaire :* | | | | |
|---|---|---|---|---|
| | *C* | *H* | *N* | *S* |
| *% théorique* | *43,90* | *4,50* | *11,38* | *13,02* |
| *% expérimental* | *43,73* | *4,37* | *11,10* | *12,80* |

### Préparation 3 : 4-(2-Chloroéthyl)-3,4-dihydro-2H-1,2,4-benzothiadiazin-7-ol 1,1-dioxide

### Stade A : 4-(2-Chloroéthyl)-7-méthoxy-4H-1,2,4-benzothiadiazine 1,1-dioxide

A une suspension de 1,0 g (3,90 mmol) du produit du stade A de la préparation 1 dans 20 ml de CH₂Cl₂ on ajoute, à température ambiante, 0,1 ml de DMF puis, goutte à goutte, une solution contenant 1,42 ml (19,5 mmol) de SOCl₂ dans 5 ml de CH₂Cl₂. A la fin de l'addition, on obtient une solution qui est agitée au reflux du CH₂Cl₂ pendant 2 h. Le CH₂Cl₂ est évaporé sous vide et le résidu est repris dans une solution 5% de NaHCO₃. Après trituration du résidu, on obtient un solide qui est filtré, rincé par de l'eau, séché pour conduire au produit du titre.

### Point de fusion : 126-130 °C

| *Microanalyse élémentaire :* | | | | |
|---|---|---|---|---|
| | *C* | *H* | *N* | *S* |
| *% théorique* | *43,72* | *4, 04* | *10, 20* | *11, 67* |
| *% expérimental* | *43,79* | *4, 06* | *9,84* | *12,01* |

### Stade B : 4-(2-Chloroéthyl)-7-méthoxy-3,4-dihydro-2H-1,2,4-benzothiadiazine 1,1-dioxide

Conditions et traitement identiques au stade C de la préparation 1.

### Point de fusion : 139-143 °C

| *Microanalyse élémentaire :* | | | | | |
|---|---|---|---|---|---|
| | *C* | *H* | *N* | *S* | *Cl* |
| *% théorique* | *43,40* | *4,73* | *10,12* | *11,59* | *12,81* |
| *% expérimental* | *43,73* | *5,05* | *9,89* | *11,07* | *13,30* |

### Stade C : 4-(2-Chloroéthyl)-3,4-dihydro-2H-1,2,4-benzothiadiazin-7-ol 1,1-dioxide

Conditions et traitement identiques au stade D de la préparation 1.

### Point de fusion : 171-173 °C

Les produits des exemples 1-12 ont été obtenus par une réaction de O-Arylation des intermédiaires décrits dans les préparations 1, 2 ou 3 avec l'acide boronique approprié et selon les conditions de réaction et de traitement décrites dans l'exemple 1 ci-dessous.

### Exemple 1 : N-(4-{[4-(2-Bromoéthyl)-1,1-dioxido-3,4-dihydro-2H-1,2,4-benzothiadiazin-7-yl]oxy}benzyl)méthanesulfonamide

Dans un erlen de 100 ml, on introduit 80 ml de CH₂Cl₂, 548 µl (6,79 mmol) de pyridine, 700 mg (2,29 mmol) du produit de la préparation 1, 8 g de tamis moléculaire 4 Å, 786 mg (3,43 mmol) d'acide 4-{[(méthylsulfonyl)amino]méthyl phényl)boronique et 623 mg (3,43 mmol) de Cu(OAc)₂. On agite vigoureusement la suspension à température ambiante en laissant l'erlen ouvert à l'air ambiant. Après 4 h 30, la réaction est diluée dans 50 ml de CH₂Cl₂ supplémentaires et la suspension est filtrée. Le filtrat est évaporé à sec et le résidu est chromatographié deux fois sur colonne de silice en éluant successivement par CH₂Cl₂/MeOH (98/2) pour la première chromatographie et CH₂Cl₂/acétone (95/5) pour la deuxième pour conduire au produit du titre.

### Point de fusion : 182-184 °C

| *Microanalyse élémentaire :* | | | | |
|---|---|---|---|---|
| | *C* | *H* | *N* | *S* |
| *% théorique* | *41,64* | *4,11* | *8,57* | *13,08* |
| *% expérimental* | *42,05* | *3,76* | *8,29* | *13,09* |

### Exemple 2 : N-(4-{[4-(2-Fluoroéthyl)-1,1-dioxido-3,4-dihydro-2H-1,2,4-benzothiadiazin-7-yl]oxy}benzyl)méthanesulfonamide

On procède comme dans l'Exemple 1, à partir du composé obtenu dans la Préparation 2 et de l'acide 4-{[(méthylsulfonyl)amino]méthyl}phényl boronique.

### Point de fusion : 100-102 °C

| *Microanalyse élémentaire :* | | | | |
|---|---|---|---|---|
| | C | *H* | *N* | *S* |
| *% théorique* | *47,54* | *4,69* | *9,78* | *14,93* |
| *% expérimental* | *47,14* | *4,97* | *9,56* | *14,99* |

### Exemple 3 : N-(4-{[4-(2-Chloroéthyl)-1,1-dioxido-3,4-dihydro-2H-1,2,4-benzothiadiazin-7-yl]oxy}benzyl)méthanesulfonamide

On procède comme dans l'Exemple 1, à partir du composé obtenu dans la Préparation 3 et de l'acide 4-{[(méthylsulfonyl)amino]méthyl}phényl boronique.

### Point de fusion : 172-175 °C

| *Microanalyse élémentaire :* | | | | | |
|---|---|---|---|---|---|
| | *C* | *H* | *N* | *S* | *Cl* |
| *% théorique* | *45,79* | *4,52* | *9,42* | *14,38* | *7,95* |
| *% expérimental* | *45,55* | *4,84* | *9,24* | *14,69* | *8,41* |

### Exemple 4 : N-(3-{[4-(2-Fluoroéthyl)-1,1-dioxido-3,4-dihydro-2H-1,2,4-benzothiadiazin-7-yl]oxy}phényl)méthanesulfonamide

On procède comme dans l'Exemple 1, à partir du composé obtenu dans la Préparation 2 et de l'acide 3-{[(méthylsulfonyl)amino]phényl boronique.

### Point defusion : 131-134 °C

| *Microanalyse élémentaire :* | | | | |
|---|---|---|---|---|
| | *C* | *H* | *N* | *S* |
| *% théorique* | *46,26* | *4,37* | *10,11* | *15,44* |
| *% expérimental* | *46,09* | *4,35* | *9,91* | *15,85* |

### Exemple 5 : N-(4-{[4-(2-Fluoroéthyl)-1,1-dioxido-3,4-dihydro-2H-1,2,4-benzothiadiazin-7-yl]oxy}phényl)méthanesulfonamide

On procède comme dans l'Exemple 1, à partir du composé obtenu dans la Préparation 2 et de l'acide 4-{[(méthylsulfonyl)amino]phényl boronique.

### Point de fusion : 151-152 °C

| *Microanalyse élémentaire :* | | | | |
|---|---|---|---|---|
| | *C* | *H* | *N* | *S* |
| *% théorique* | *46,26* | *4,37* | *10,11* | *15,44* |
| *% expérimental* | *45,71* | *4,78* | *9,90* | *15,65* |

### Exemple 6 : 4-(2-Fluoroéthyl)-7-(3-méthoxyphénoxy)-3,4-dihydro-2H-1,2,4-benzothiadiazine 1,1-dioxide

On procède comme dans l'Exemple 1, à partir du composé obtenu dans la Préparation 2 et de l'acide 3-méthoxyphényl boronique.

### Point de fusion : 101-102 °C

| *Microanalyse élémentaire :* | | | | |
|---|---|---|---|---|
| | *C* | *H* | *N* | *S* |
| *% théorique* | *54,54* | *4,86* | *7,95* | *9,10* |
| *% expérimental* | *54,50* | *4,85* | *7,77* | *8,91* |

### Exemple 7 : N-(3-{[4-(2-Fluoroéthyl)-1,1-dioxido-3,4-dihydro-2H-1,2,4-benzothiadiazin-7-yl]oxy}benzyl)acétamide

On procède comme dans l'Exemple 1, à partir du composé obtenu dans la Préparation 2 et de l'acide 3-[(acétylamino)méthyl]phényl boronique.

### Point de fusion : 129-131 °C

| *Microanalyse élémentaire :* | | | | |
|---|---|---|---|---|
| | *C* | *H* | *N* | *S* |
| *% théorique* | *54,95* | *5,12* | *10,68* | *8,15* |
| *% expérimental* | *55,03* | *5,18* | *10,35* | *8,22* |

### Exemple 8 : N-(3-{[4-(2-Fluoroéthyl)-1,1-dioxido-3,4-dihydro-2H-1,2,4-benzothiadiazin-7-yl]oxy}benzyl)méthanesulfonamide

On procède comme dans l'Exemple 1, à partir du composé obtenu dans la Préparation 2 et de l'acide 3-{[(méthylsulfonyl)amino]méthyl}phényl boronique.

### Point de fusion : 110-112 °C

| *Microanalyse élémentaire :* | | | | |
|---|---|---|---|---|
| | *C* | *H* | *N* | *S* |
| *% théorique* | *45,54* | *4, 69* | *9,78* | *14,93* |
| *% expérimental* | *47,26* | *4,86* | *9,45* | *15,01* |

### Exemple 9 : N-(4-{[4-(2-Fluoroéthyl)-1,1-dioxido-3,4-dihydro-2H-1,2,4-benzothiadiazin-7-yl]oxy}benzyl)-N-méthylméthanesulfonamide

On procède comme dans l'Exemple 1, à partir du composé obtenu dans la Préparation 2 et de l'acide 4-{[méthyl(méthylsulfonyl)amino]méthyl}phényl) boronique.

### Point de fusion : 59 °C

| *Microanalyse élémentaire :* | | | | |
|---|---|---|---|---|
| | *C* | *H* | *N* | *S* |
| *% théorique* | *48,75* | *5,00* | *9,47* | *14,46* |
| *% expérimental* | *48,53* | *5,16* | *9,06* | *14,41* |

### Exemple 10 : 4-(2-Fluoroéthyl)-7-phénoxy-3,4-dihydro-2H-1,2,4-benzothiadiazine 1,1-dioxide

On procède comme dans l'Exemple 1, à partir du composé obtenu dans la Préparation 2 et de l'acide phényl boronique.

### Point de fusion : 145 °C

| *Microanalyse élémentaire :* | | | | |
|---|---|---|---|---|
| | *C* | *H* | *N* | *S* |
| *% théorique* | *55,89* | *4, 69* | *8,69* | *9,95* |
| *% expérimental* | *55,70* | *4,81* | *8,48* | *9,89* |

### Exemple 11 : 3-{[4-(2-Fluoroéthyl)1,1-dioxido-3,4-dihydro-2H-1,2,4-benzothiadiazin-7-yl]oxy}-N'-hydroxybenzenecarboximidamide

### Stade A : 3-{[4-(2-Fluoroéthyl)-1,1-dioxido-4H-1,2,4-benzothiadiazin-7-yl]oxy}benzonitrile

On procède, comme dans l'Exemple 1, à partir du composé obtenu au stade A de la Préparation 2 et de l'acide 3-cyanophénylboronique et le temps de réaction est porté à 48 h.

### Point de fusion : 202-208 °C

### Stade B : 3-{[4-(2-Fluoroéthyl)-1,1-dioxido-4H-1,2,4-benzothiadiazin-7-yl]oxy}-N'-hydroxybenzenecarboximidamide

A une solution du chlorhydrate d'hydroxylamine (384 mg, 5,52 mmol) dans 1,8 ml de DMSO sont ajoutés 770 µL (5,52 mmol) de triéthylamine et la suspension est agitée 20 min à température ambiante. Le précipité est filtré et le filtrat concentré sous vide. A ce filtrat sont rajoutés 225 mg (0,921 mmol) du produit du stade A précédent et la solution est agitée à 75 °C pendant 4 h. La réaction est refroidie à température ambiante et le milieu réactionnel est précipité dans l'eau. Une pâte gommeuse blanche non filtrable est obtenue que l'on sépare de la phase aqueuse par simple décantation. La gomme est triturée dans l'éthanol jusqu'à cristallisation. Le solide est filtré pour conduire au produit du titre.

### Point de fusion : 181-183 °C

### Stade C : 3-{[4-(2-Fluoroéthyl)-1,1-dioxido-3,4-dihydro-2H-1,2,4-benzothiadiazin-7-yl]oxy}-N'-hydroxybenzènecarboximidamide

A une suspension du produit obtenu au stade B (75 mg, 0,198 mmol) dans 1 ml d'éthanol sont ajoutés 10 mg (0,258 mmol) de NaBH₄. La suspension est agitée 30 min, à température ambiante, puis refroidie dans un bain de glace. Le milieu réactionnel est neutralisé par addition de HCl 1N et extrait par de l'acétate d'éthyle. La phase organique est lavée par NaCl saturé, séchée (MgSO₄), évaporée sous vide. Le résidu d'évaporation est cristallisé dans du CH₂Cl₂. Le produit attendu est récupéré par filtration.

### Point de fusion : 160-163 °C

### Exemple 12 : 3-{[4-(2-Fluoroéthyl)-1,1-dioxido-3,4-dihydro-2H-1,2,4-benzothiadiazin-7-yl]oxy)-N-méthylbenzamide

On procède, comme dans l'Exemple 1, à partir du composé obtenu dans la Préparation 2 et de l'acide 3-[(méthylamino)carbonyl]phényl boronique.

### Point de fusion : 194-196 °C

| *Microanalyse élémentaire :* | | | | |
|---|---|---|---|---|
| | *C* | *H* | *N* | *S* |
| *% théorique* | *53,82* | *4,78* | *11, 08* | *8,45* |
| *% expérimental* | *53,53* | *4,98* | *10,87* | *8,42* |

### ETUDE PHARMACOLOGIQUE DES PRODUITS DE L'INVENTION

### Etude des courants excitateurs induits par l'AMPA dans les oocytes de Xenopus

### a - Méthode :

Des ARNm sont préparés à partir de cortex cérébral de rat mâle Wistar par la méthode au guanidium thiocyanate/phénol/chloroforme. Les ARNm Poly (A⁺) sont isolés par chromatographie sur oligo-dT cellulose et injectés à raison de 50 ng par oocyte. Les oocytes sont laissés 2 à 3 jours en incubation à 18°C pour permettre l'expression des récepteurs puis stockés à 8-10°C.
L'enregistrement électrophysiologique est réalisé dans une chambre en plexiglass® à 20-24°C en milieu OR2 (J. Exp. Zool., 1973, 184, 321-334) par la méthode du «voltage-clamp» à 2 électrodes, une 3^{ème} électrode placée dans le bain servant de référence.

Tous les composés sont appliqués via le milieu d'incubation et le courant électrique est mesuré à la fin de la période d'application. L'AMPA est utilisé à la concentration de 10 µM. Pour chaque composé étudié, on définit la concentration doublant (EC2X) ou quintuplant (EC5X) l'intensité du courant induit par l'AMPA seul (5 à 50 nA).

### b- Résultats :

Les composés de l'invention potentialisent très fortement les effets excitateurs de l'AMPA et leur activité est très nettement supérieure à celle des composés de référence.

A titre d'exemple, le composé de l'Exemple 1 présente une EC2X de 0,04 µM.

### COMPOSITION PHARMACEUTIQUE

| | |
|---|---|
| Formule de préparation pour 1000 comprimés dosés à 100 mg de *N*-(4-{[4-(2-Fluoroéthyl)-1,1-dioxido-3,4-dihydro-2*H*-1,2,4-benzothiadiazin-7-yl]oxy}benzyl)méthanesulfonamide (exemple 2) | 100 g |
| Hydroxypropycellulose | 2 g |
| Amidon de blé | 10 g |
| Lactose | 100 g |
| Stéarate de magnésium | 3 g |
| Talc | 3 g |

## Revendications

1. Composés de formule (I) : 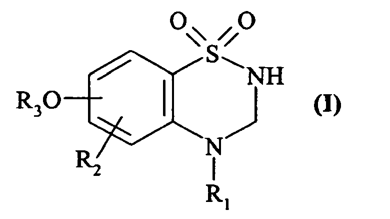 dans laquelle :
**R₁** représente un groupement alkyle (C₁-C₆) linéaire ou ramifié substitué par un ou plusieurs atomes d'halogène,
**R₂** représente un atome d'hydrogène ou d'halogène ou un groupement hydroxy,
**R₃** représente un groupement aryle non substitué ou un groupement aryle substitué par un ou plusieurs groupements, identiques ou différents, choisis parmi :
alkyle (C₁-C₆) linéaire ou ramifié ; alkoxy (C₁-C₆) linéaire ou ramifié ; polyhalogénoalkyle (C₁-C₆) linéaire ou ramifié ; atomes d'halogène ; alkoxycarbonyle (C₁-C₆) linéaire ou ramifié ; alkylthio (C₁-C₆) linéaire ou ramifié ; carboxy ; acyle (C₁-C₆) linéaire ou ramifié ; polyhalogénoalkoxy (C₁-C₆) linéaire ou ramifié ; hydroxy ; cyano ; nitro ; amidino (éventuellement substitué par un ou deux groupements identiques ou différents choisis parmi alkyle (C₁-C₆) linéaire ou ramifié, hydroxy, alkoxy (C₁-C₆) linéaire ou ramifié, et 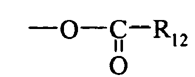 ) ; amino (éventuellement substitué par un ou deux groupements alkyle (C₁-C₆) linéaire ou ramifié) ; aminocarbonyle (éventuellement substitué par un ou deux groupements alkyle (C₁-C₆) linéaire ou ramifié) ; benzyloxy ; alkyl (C₁-C₆) sulfonylamino (éventuellement substitué sur l'azote par un groupement (C₁-C₆) linéaire ou ramifié) ; (trifluorométhylsulfonyl)amino ; groupement hétérocyclique ; et alkyle (C₁-C₆) linéaire ou ramifié substitué d'une part par un ou plusieurs groupements, identiques ou différents, choisis parmi les atomes d'hydrogène ou d'halogène et le groupement alkyle (C₁-C₆) linéaire ou ramifié, et substitué d'autre part par un groupement choisi parmi NR₄R₅, S(O)ₙR₆, OR₇, amidino (éventuellement substitué par un ou deux groupements identiques ou différents choisis parmi alkyle (C₁-C₆) linéaire ou ramifié, hydroxy, alkoxy (C₁-C₆) linéaire ou ramifié, et 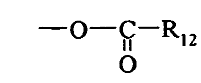 ), et groupement hétérocyclique, dans lesquels :
R₄ représente un atome d'hydrogène, ou un groupement alkyle (C₁-C₆) linéaire ou ramifié, S(O)ₚR₈, COR₉ ou P(O)(OR₁₀)(OR₁₁),
R₅ représente un atome d'hydrogène, ou un groupement alkyle (C₁-C₆) linéaire ou ramifié,
ou bien R₄ et R₅ forment ensemble, avec l'atome d'azote qui les porte, un groupement hétérocyclique,
R₆, R₈, R₉, R₁₀, R₁₁ et R₁₂ identiques ou différents, représentent un atome d'hydrogène ou un groupement alkyle (C₁-C₆) linéaire ou ramifié éventuellement substitué par un ou plusieurs atomes d'halogène ; un groupement arylalkyle (C₁-C₆) linéaire ou ramifié ; ou un groupement aryle,
R₇ représente un groupement alkyle (C₁-C₆) linéaire ou ramifié ou acyle (C₁-C₆) linéaire ou ramifié,
n et p, identiques ou différents, représentent 0, 1 ou 2,
leur énantiomères et diastéréoisomères ainsi que leurs sels d'addition à un acide ou a une base pharmaceutiquement acceptable.

2. Composés de formule (I), selon la revendication 1, pour lesquels R₁ représente un groupement halogénoéthyle.

3. Composés de formule (I), selon la revendication 1, pour lesquels R₂ représente un atome d'hydrogène.

4. Composés de formule (I), selon la revendication 1, pour lesquels R₃ représente un groupement phényle non substitué.

5. Composés de formule (I), selon la revendication 1, pour lesquels R₃ représente un groupement phényle substitué par un groupement amidino, hydroxyamidino, alkoxy, alkylsulfonylamino (éventuellement substitué sur l'azote par un groupement alkyle), ou alkyle substitué par un groupement amidino, hydroxyamidino, OR₇, NH(SO)ₚR₈ ou NHCOR₉.

6. Composés de formule (I), selon la revendication 1, qui sont le :
• le *N*-(4-{[4-(2-fluoroéthyl)-1,1-dioxido-3,4-dihydro-2*H*-1,2,4-benzothiadiazin-7-yl]oxy}benzyl)méthanesulfonamide,
• et le *N*-(4-{[4-(2-chloroéthyl)-1,1-dioxido-3,4-dihydro-2*H*-1,2,4-benzothiadiazin-7-yl]oxy}benzyl)méthanesulfonamide.

7. Procédé de préparation des composés de formule (I), selon la revendication 1, **caractérisé en ce que** l'on utilise comme produit de départ le composé de formule (II) : 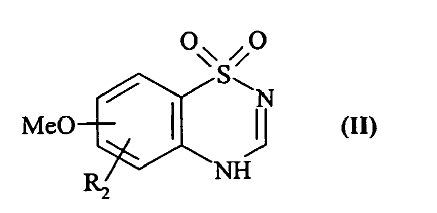 dans laquelle R₂ est tel que défini dans la formule (I),
sur lequel on condense, en milieu basique, un halogénoalkyle (C₁-C₆) linéaire ou ramifié comportant une fonction hydroxyle,
que l'on transforme ensuite en dérivé halogéné correspondant pour conduire au composé de formule (III) : 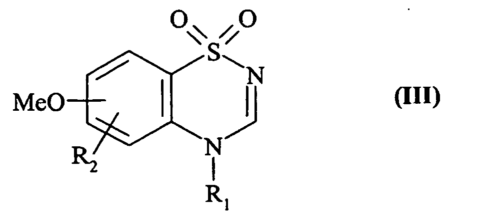 dans laquelle R₁ et R₂ sont tels que définis dans la formule (I),
➢ que l'on soumet à une réaction de déméthylation, en présence de BBr₃ ou BF₃ par exemple, pour conduire au composé de formule (IV) : 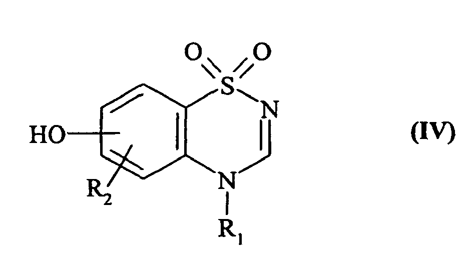 dans laquelle R₁ et R₂ sont tels que définis précédemment,
sur lequel on condense, en présence de Cu(OAc)₂, le dérivé boronique de formule (V) :
R₃-B(OH)₂ (V)
dans laquelle R₃ est tel que défini dans la formule (I),
pour conduire au composé de formule (VI) : 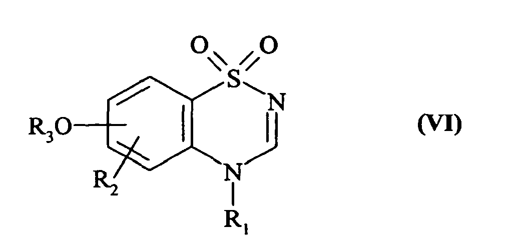 dans laquelle R₁, R₂ et R₃ sont tels que définis précédemment,
que l'on soumet à une réduction avec NaBH₄ par exemple, pour conduire au composé de formule (I) : 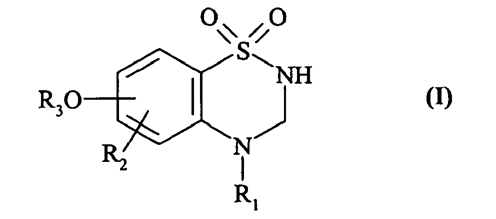 dans laquelle R₁, R₂ et R₃ sont tels que définis précédemment,
➢ ou composé et formule (III) que l'on soumet à une réduction, en présence de NaBH₄ par exemple, pour obtenir le composé de formule (VII) : 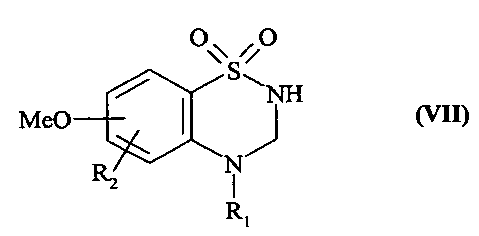 dans laquelle R₁ et R₂ sont tels que définis précédemment,
que l'on soumet à une réaction de déméthylation, en présence de BBr₃ ou BF₃ par exemple, pour conduire au composé de formule (VIII) : 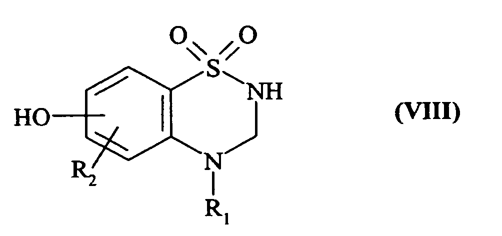 dans laquelle R₁ et R₂ sont tels que définis précédemment,
sur lequel on condense, en présence de Cu(OAc)₂, le dérivé boronique de formule (V) tel que défini précédemment pour conduire au composé et formule (I),
composé de formule (I) que l'on purifie, le cas échéant, selon une technique classique de purification, dont on sépare éventuellement les isomères, selon une technique classique de séparation, et que l'on transforme, si on le souhaite, en leurs sels d'addition, à un acide ou à une base pharmaceutiquement acceptable.

8. Composés de formule (VIII) selon la revendication 7 : 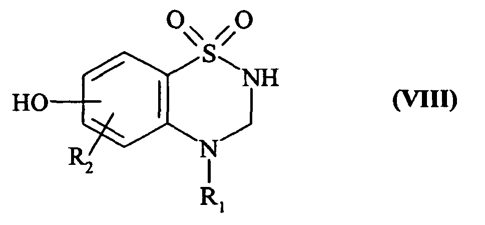 dans laquelle R₁ et R₂ sont tels que définis dans la revendication 7, utiles en tant qu'intermédiaires de synthèse des composés de formule (I).

9. Composés de formule (IX) cas particulier des composés de formule (VIII) selon la revendication 8 : 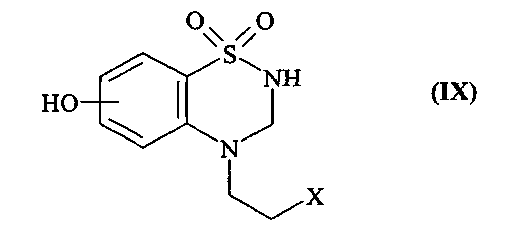 dans laquelle X représente un atome de fluor, de chlore, de brome ou d'iode, utiles en tant qu'intermédiaires de synthèse des composés de formule (I).

10. Compositions pharmaceutiques contenant comme principe actif un composé selon l'une quelconque des revendications 1 à 6, 8 et 9 en combinaison avec un ou plusieurs excipients ou véhicules inertes, non toxiques, pharmaceutiquement acceptables.

11. Compositions pharmaceutiques selon la revendication 10 contenant comme principe actif un composé selon l'une quelconque des revendications 1 à 6, 8 et 9 utiles en tant que médicaments, comme modulateurs AMPA.

## Claims

1. Compounds of formula (I) : 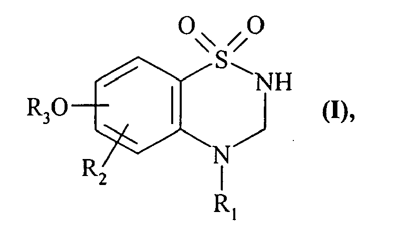 wherein :
**R₁** represents a linear or branched (C₁-C₆)alkyl group substituted by one or more halogen atoms,
**R₂** represents a hydrogen atom, a halogen atom or a hydroxy group,
**R₃** represents an unsubstituted aryl group or an aryl group substituted by one or more identical or different groups selected from :
linear or branched (C₁-C₆)alkyl; linear or branched (C₁-C₆)alkoxy; linear or branched (C₁-C₆)polyhaloalkyl; halogen atoms; linear or branched (C₁-C₆)alkoxy-carbonyl; linear or branched (C₁-C₆)alkylthio; carboxy; linear or branched (C₁-C₆)acyl; linear or branched (C₁-C₆)polyhaloalkoxy; hydroxy; cyano; nitro; amidino (optionally substituted by one or two identical or different groups selected from linear or branched (C₁-C₆)alkyl, hydroxy, linear or branched (C₁-C₆)alkoxy and 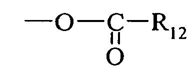 ); amino (optionally substituted by one or two linear or branched (C₁-C₆)alkyl groups); aminocarbonyl (optionally substituted by one or two linear or branched (C₁-C₆)alkyl groups); benzyloxy; (C₁-C₆)alkylsulphonylamino (optionally substituted on the nitrogen by a linear or branched (C₁-C₆) group); (trifluoromethylsulphonyl)amino; a heterocyclic group; and linear or branched (C₁-C₆)alkyl on the one hand substituted by one or more identical or different groups selected from hydrogen and halogen atoms and linear or branched (C₁-C₆)alkyl groups and on the other hand substituted by a group selected from NR₄R₅, S(O)ₙR₆, OR₇, amidino (optionally substituted by one or two identical or different groups selected from linear or branched (C₁-C₆)alkyl, hydroxy, linear or branched (C₁-C₆)alkoxy and 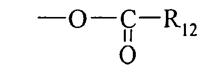 ), and a heterocyclic group, wherein :
R₄ represents a hydrogen atom or a linear or branched (C₁-C₆)alkyl, S(O)ₚR₈, COR₉ or P(O)(OR₁₀)(OR₁₁) group,
R₅ represents a hydrogen atom or a linear or branched (C₁-C₆)alkyl group,
or R₄ and R₅, together with the nitrogen atom carrying them, form a heterocyclic group, R₆, R₈, R₉, Rio, R₁₁ and R₁₂, which may be the same or different, each represent a hydrogen atom or a linear or branched (C₁-C₆)alkyl group optionally substituted by one or more halogen atoms; an aryl-(C₁-C₆)alkyl group in which the alkyl moiety is linear or branched; or an aryl group,
R₇ represents a linear or branched (C₁-C₆)alkyl group or a linear or branched (C₁-C₆)acyl group,
n and p, which may be the same or different, each represent 0, 1 or 2,
their enantiomers and diastereoisomers, and addition salts thereof with a pharmaceutically acceptable acid or base.

2. Compounds of formula (I), according to claim 1, wherein R₁ represents a haloethyl group.

3. Compounds of formula (I), according to claim 1, wherein R₂ represents a hydrogen atom.

4. Compounds of formula (I), according to claim 1, wherein R₃ represents an unsubstituted phenyl group.

5. Compounds of formula (I), according to claim 1, wherein R₃ represents a phenyl group substituted by an amidino group, by a hydroxyamidino group, by an alkoxy group, by an alkylsulphonylamino group (optionally substituted on the nitrogen by an alkyl group), or by an alkyl group substituted by an amidino, hydroxyamidino, OR₇, NH(SO)ₚR₈ or NHCOR₉ group.

6. Compounds of formula (I), according to claim 1, which are :
• *N*-(4-{[4-(2-fluoroethyl)-1,1-dioxido-3,4-dihydro-2*H*-1,2,4-benzothiadiazin-7-yl]oxy}benzyl)methanesulphonamide,
• and *N*-(4-{[4-(2-chloroethyl)-1,1-dioxido-3,4-dihydro-2*H*-1,2,4-benzothiadiazin-7-yl]oxy} benzyl)methanesulphonamide.

7. Process for the preparation of compounds of formula (I), according to claim 1, **characterised in that** there is used as starting material the compound of formula (II) : 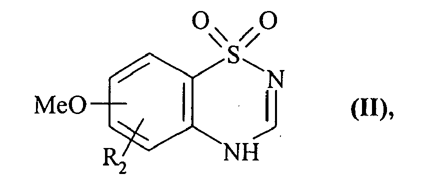 wherein R₂ is as defined for formula (I),
with which there is condensed, in a basic medium, a linear or branched (C₁-C₆)haloalkyl having a hydroxyl function,
which is then converted into a corresponding halogenated compound to yield the compound of formula (III) : 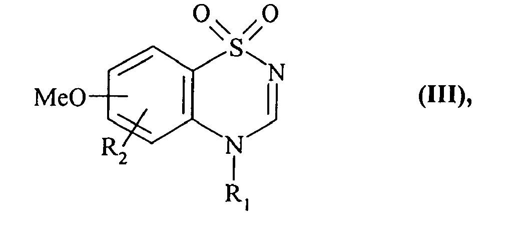 wherein R₁ and R₂ are as defined for formula (I),
➢ which is subjected to a demethylation reaction, in the presence of BBr₃ or BF₃, for example, to yield the compound of formula (IV) : 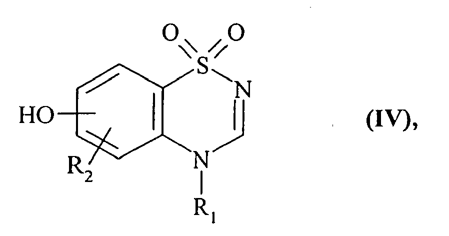 wherein R₁ and R₂ are as defined hereinbefore,
with which there is condensed, in the presence of Cu(OAc)₂, the boron compound of formula (V) :
R₃-B(OH)₂ (V),
wherein R₃ is as defined for formula (I),
to yield the compound of formula (VI) : 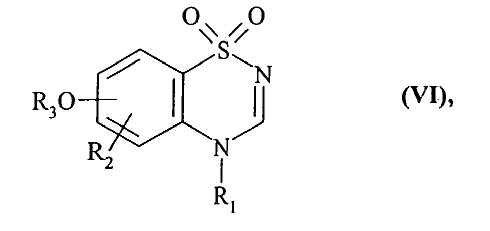 wherein R₁, R₂ and R₃ are as defined hereinbefore,
which is subjected to reduction with NaBH₄, for example, to yield the compound of formula (I) : 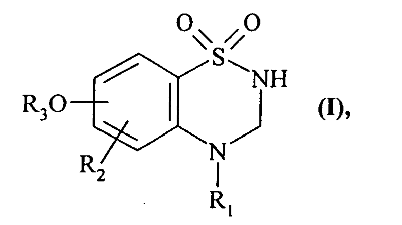 wherein R₁, R₂ and R₃ are as defined hereinbefore,
➢ or which compound of formula (III) is subjected to reduction, in the presence of NaBH₄, for example, to obtain the compound of formula (VII) : 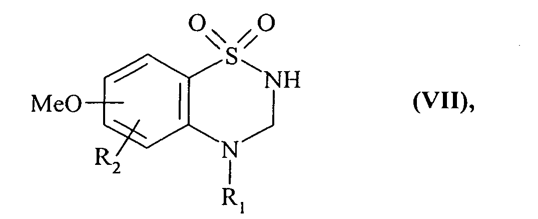 wherein R₁ and R₂ are as defined hereinbefore,
which is subjected to a demethylation reaction, in the presence of BBr₃ or BF₃, for example, to yield the compound of formula (VIII) : 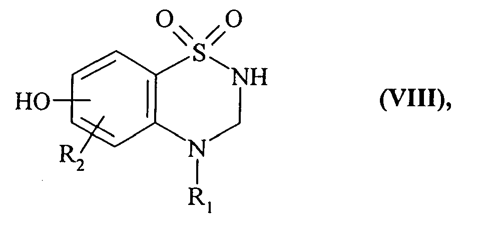 wherein R₁ and R₂ are as defined hereinbefore,
with which there is condensed, in the presence of Cu(OAc)₂, the boron compound of formula (V) as defined hereinbefore to yield the compound of formula (I),
which compound of formula (I) is purified, if necessary, according to a conventional purification technique, is separated, where appropriate, into its isomers according to a conventional separation technique and is converted, if desired, into its addition salts with a pharmaceutically acceptable acid or base.

8. Compounds of formula (VIII) according to claim 7 : 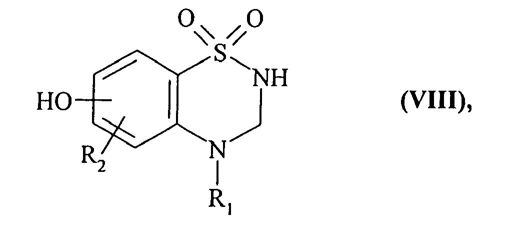 wherein R₁ and R₂ are as defined in claim 7, for use as synthesis intermediates for compounds of formula (I).

9. Compounds of formula (IX), a particular case of the compounds of formula (VIII) according to claim 8 : 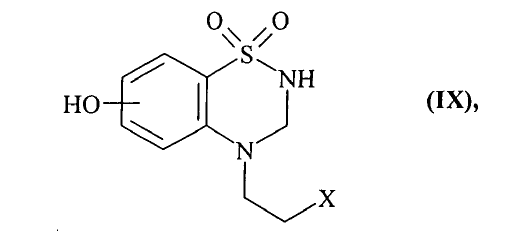 wherein X represents a fluorine, chlorine, bromine or iodine atom, for use as synthesis intermediates for compounds of formula (I).

10. Pharmaceutical compositions comprising as active ingredient a compound according to any one of claims 1 to 6, 8 and 9 in combination with one or more inert, non-toxic, pharmaceutically acceptable excipients or carriers.

11. Pharmaceutical compositions according to claim 10 comprising as active ingredient a compound according to any one of claims 1 to 6, 8 and 9, for use as medicaments as AMPA modulators.

## Patentansprüche

1. Verbindungen der Formel (I): 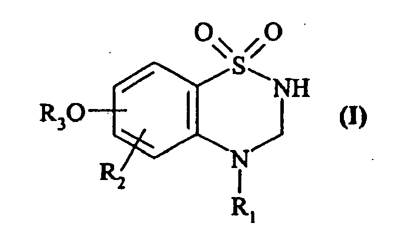 in der:
**R₁** eine geradkettige oder verzweigte, gegebenenfalls durch ein oder mehrere Halogenatome substituierte (C₁-C₆)-Alkylgruppe bedeutet,
**R₂** ein Wasserstoffatom, ein Halogenatom oder eine Hydroxygruppe bedeutet,
**R₃** eine nichtsubstituierte Arylgruppe oder eine Arylgruppe, die durch einen oder mehrere gleichartige oder verschiedenartige Substituenten substituiert ist, ausgewählt aus:
geradkettigem oder verzweigtem (C₁-C₆)-Alkyl; geradkettigem oder verzweigtem (C₁-C₆)-Alkoxy; geradkettigem oder verzweigtem (C₁-C₆)-Polyhalogenalkyl; Halogenatomen; geradkettigem oder verzweigtem (C₁-C₆)-Alkoxycarbonyl; geradkettigem oder verzweigtem (C₁-C₆)-Alkylthio; Carboxy; geradkettigem oder verzweigtem (C₁-C₆)-Acyl; geradkettigem oder verzweigtem (C₁-C₆)-Polyhalogenalkoxy; Hydroxy; Cyano; Nitro; Amidino (gegebenenfalls substituiert durch eine oder zwei gleichartige oder verschiedenartige Gruppen ausgewählt aus geradkettigem oder verzweigtem (C₁-C₆)-Alkyl, Hydroxy, geradkettigem oder verzweigtem (C₁-C₆)-Alkoxy und 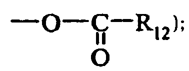 Amino (gegebenenfalls substituiert durch eine oder zwei geradkettige oder verzweigte (C₁-C₆)-Alkylgruppen); Aminocarbonyl (gegebenenfalls substituiert durch eine oder zwei geradkettige oder verzweigte (C₁-C₆)-Alkylgruppen); Benzyloxy; (C₁-C₆)-Alkylsulfonylamino (gegebenenfalls am Stickstoff substituiert durch eine geradkettige oder verzweigte (C₁-C₆)-Alkylgruppe); (Trifluormethylsulfonyl)-amino; heterocyclische Gruppen; und geradkettigem oder verzweigtem (C₁-C₆)-Alkyl, welches einerseits durch eine oder mehrere, gleichartige oder verschiedenartige Gruppen substituiert ist, ausgewählt aus Wasserstoff- oder Halogenatomen und geradkettigen oder verzweigten (C₁-C₆)-Alkylgruppen, und andererseits substituiert ist durch eine Gruppe ausgewählt aus NR₄R₅, S(O)ₙR₆, OR₇, Amidino (gegebenenfalls substituiert durch eine oder zwei gleichartige oder verschiedenartige Gruppen ausgewählt aus geradkettigem oder verzweigtem (C₁-C₆)-Alkyl, Hydroxy, geradkettigem oder verzweigtem (C₁-C₆)-Alkoxy und 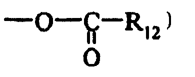 und heterocyclischen Gruppen, worin:
R₄ ein Wasserstoffatom, eine geradkettige oder verzweigte (C₁-C₆)-Alkylgruppe oder eine Gruppe S(O)ₚR₈, COR₉ oder P(O)(OR₁₀)(OR₁₁) darstellt,
R₅ ein Wasserstoffatom oder eine geradkettige oder verzweigte (C₁-C₆)-Alkylgruppe darstellt
oder R₄ und R₅ gemeinsam mit dem sie tragenden Stickstoffatom eine heterocyclische Gruppe bilden,
R₆, R₈, R₉, R₁₀, R₁₁ und R₁₂, die gleichartig oder verschieden sind, ein Wasserstoffatom, geradkettige oder verzweigte, gegebenenfalls durch Halogenatome substituierte (C₁-C₆)-Alkylgruppen; geradkettige oder verzweigte Aryl-(C₁-C₆)-alkylgruppen; oder Arylgruppen bedeuten,
R₇ eine geradkettige oder verzweigte (C₁-C₆)-Alkylgruppe oder eine geradkettige oder verzweigte (C₁-C₆)-Acylgruppe bedeutet und
n und p, die gleichartig oder verschieden sind, 0, 1 oder 2 darstellen,
deren Enantiomere, Diastereoisomere sowie deren Additionssalze mit einer pharmazeutisch annehmbaren Säure oder Base.

2. Verbindungen der Formel (I) nach Anspruch 1, worin R₁ eine Halogenethylgruppe bedeutet.

3. Verbindungen der Formel (I) nach Anspruch 1, worin R₂ ein Wasserstoffatom bedeutet.

4. Verbindungen der Formel (I) nach Anspruch 1, worin R₃ eine nicht substituierte Phenylgruppe bedeutet.

5. Verbindungen der Formel (I) nach Anspruch 1, worin R₃ eine Phenylgruppe bedeutet, die durch eine Amidinogruppe, Hydroxyamidinogruppe, Alkoxygruppe, Alkylsulfonylaminogruppe (die gegebenenfalls am Stickstoff durch eine Alkylgruppe substituiert ist) oder durch eine Amidinogruppe, Hydroxyamidinogruppe, eine Gruppe OR₇, NH(SO)ₚR₈ oder NHCOR₉ substituierte Alkylgruppe substituiert ist.

6. Verbindungen der Formel (I) nach Anspruch 1, nämlich:
• *N*-(4-{[4-(2-Fluorethyl)-1,1-dioxido-3,4-dihydro-2*H*-1,2,4-benzothiadiazin-7-yl]-oxy}-benzyl)-methansulfonamid und
• *N*-(4-{[4-(2-Chlorethyl)-1,1-dioxido-3,4-dihydro-2*H*-1,2,4-benzothiadiazin-7-yl]-oxy}-benzyl)-methansulfonamid.

7. Verfahren zur Herstellung der Verbindungen der Formel (I) nach Anspruch 1, **dadurch gekennzeichnet, daß** man als Ausgangsprodukt die Verbindung der Formel (II) verwendet: 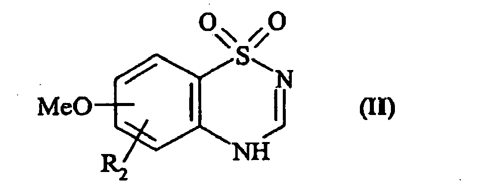 in der R₂ die bezüglich der Formel (I) angegebenen Bedeutungen besitzt, welche man in basischem Medium mit einem geradkettigen oder verzweigten (C₁-C₆)-Halogenalkyl, das eine Hydroxylfunktion aufweist, kondensiert, anschließend in das entsprechende Halogenderivat umwandelt zur Bildung der Verbindung der Formel (III): 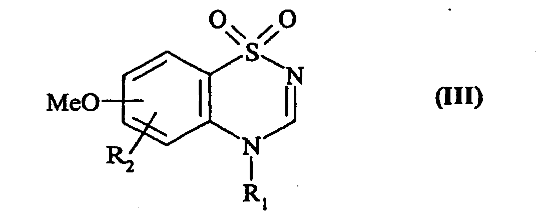 in der R₁ und R₂ die bezüglich der Formel (I) angegebenen Bedeutungen besitzen,
➢ welche man einer Demethylierungsreaktion unterwirft, beispielsweise in Gegenwart von BBr₃ oder BF₃, zur Bildung der Verbindung der Formel (IV): 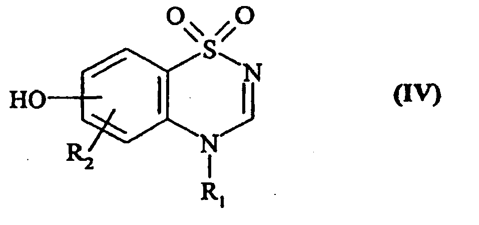 in der R₁ und R₂ die oben angegebenen Bedeutungen besitzen,
welche man in Gegenwart von Cu(OAc)₂ mit dem Boronsäurederivat der Formel (V) kondensiert:
R₃ - B(OH)₂ (V)
in der R₃ die bezüglich der Formel (I) angegebenen Bedeutungen besitzt,
zur Bildung der Verbindung der Formel (VI): 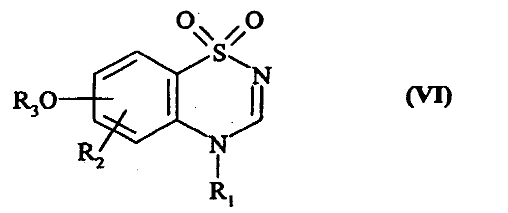 in der R₁, R₂ und R₃ die oben angegebenen Bedeutungen besitzen,
welche man einer Reduktion mit beispielsweise NaBH₄ unterwirft zur Bildung der Verbindung der Formel (I): 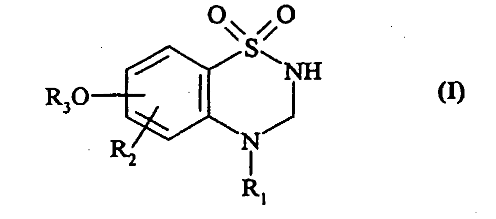 in der R₁, R₂ und R₃ die oben angegebenen Bedeutungen besitzen,
➢ - oder welche Verbindung der Formel (III) man einer Reduktion in Gegenwart von beispielsweise NaBH₄ unterwirft zur Bildung der Verbindung der Formel (VII): 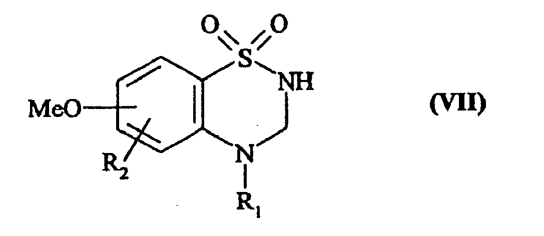 in der R₁ und R₂ die oben angegebenen Bedeutungen besitzen,
die man einer Demethylierungsreaktion unterwirft, beispielsweise in Gegenwart von BBr₃ oder BF₃, zur Bildung der Verbindung der Formel (VIII): 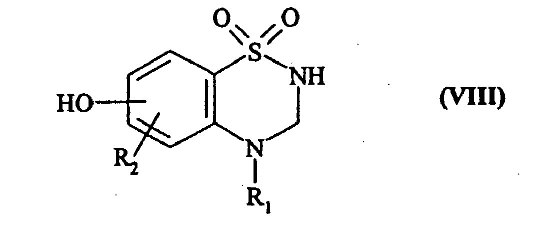 in der R₁ und R₂ die oben angegebenen Bedeutungen besitzen,
welche man in Gegenwart von Cu(OAc)₂ mit dem Boronsäurederivat der oben definierten Formel (V) kondensiert zur Bildung der Verbindung der Formel (I),
welche man gegebenenfalls mit Hilfe einer klassischen Reinigungsmethode reinigt, welche man gegebenenfalls mit Hilfe einer klassischen Trennmethode in ihre Isomeren auftrennt und welche man gewünschtenfalls in ihre Additionssalze mit einer pharmazeutisch annehmbaren Säure oder Base überführt.

8. Verbindungen der Formel (VIII) nach Anspruch 7: 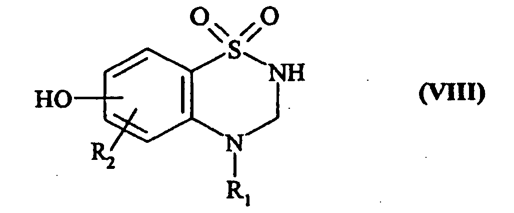 in der R₁ und R₂ die in Anspruch 7 angegebenen Bedeutungen besitzen, nützlich als Zwischenprodukte für die Synthese der Verbindungen der Formel (I).

9. Verbindungen der Formel (IX), ein Sonderfall der Verbindungen der Formel (VIII) nach Anspruch 8: 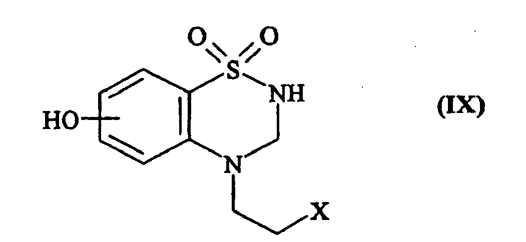 in der X ein Fluor-, Chlor-, Brom oder Iodatom bedeutet, nützlich als Zwischenprodukte bei der Synthese der Verbindungen der Formel (I).

10. Pharmazeutische Zubereitungen, enthaltend als Wirkstoff eine Verbindung nach einem der Ansprüche 1 bis 6, 8 und 9 in Kombination mit einem oder mehreren inerten, nichttoxischen, pharmazeutisch annehmbaren Trägermaterialien oder Hilfsstoffen.

11. Pharmazeutische Zubereitungen nach Anspruch 10, enthaltend als Wirkstoff eine Verbindung nach einem der Ansprüche 1 bis 6, 8 und 9, nützlich als Arzneimittel, namentlich AMPA-Modulatoren.
